# EUROPEAN PATENT SPECIFICATION

(11) **EP 0 700 719 B1**
(45) Date of publication and mention of the grant of the patent: **03.03.1999**
(21) Application number: 95114120.9
(22) Date of filing: 08.09.1995
(51) Int. Cl.: B01L 3/14, G06K 19/077, G06K 7/08, G01N 35/00

(54) **Automatic measuring equipment for measuring physical properties of a cellulose derivative solution**
Automatische Messvorrichtung zur Messung der physikalischen Eigenschaften einer Zellstoff-derivatlösung
Appareil automatique de mesure de proprietés physiques de solutions de derivés de cellulose

(30) Priority: 09.09.1994 JP 215459/94
(43) Date of publication of application: 13.03.1996
(73) Proprietor: SHIN-ETSU CHEMICAL CO., LTD., Chiyoda-ku Tokyo (JP)
(72) Inventor: Miyao, Shuichi, Naoetsu Fact. Shin-Etsu Chemical, Nakakubiki-gun, Niigata 942-01 (JP)
(74) Representative: Tiedtke, Harro, Dipl.-Ing.

(56) References cited:
- EP-A- 0 317 325
- EP-A- 0 606 121
- WO-A-89/08264
- WO-A-94/15219
- DE-A- 4 306 563
- FR-A- 2 555 744
- CHEMOMETRICS AND INTELLIGENT LABORATORY SYSTEMS, vol. 17, no. 1, 1 October 1992 AMSTERDAM NL, pages 123-127, XP 000321835 LOVERIDGE M C ET AL 'ROBOTIC SAMPLE PREPARATION UNATTENDED RECONSTITUTION AND ANALYSIS OF DRY POWDER PRESENTATIONS'
- J.GENTSCH,"FLEXIBLE LABORATORY AUTOMATION",CHEMOMETRICS & INTELLIGENT LABORATORY SYSTEMS:LABORATORY INFORMATION MANAGEMENT,21(1993) PP 229-233

## Description

The present invention relates to an automatic measuring equipment for measuring physical properties of a cellulose derivative solution according to claim 1.

The cellulose derivatives are manufactured into granular powder, and the products thus made undergo a variety of physical property tests for every production lot. The items of the physical property tests have been increasing due to diversification of product types and that of their uses. The inspection items involve specimens inspected as granular powder and those inspected as solution of the granular powder. The automatization of the inspections has been under discussion. The inspection items for solution include, for instance, measurement of viscosity, electrochemical analysis such as inorganic analysis and measurement, measurement of transmittance such as absorbance of parallel rays and scattered light, and measurement of image such as non-dissolved fiber component.

The cellulose derivatives are apt to be statically electrified. Since moreover the powdered derivatives in the form of fibrous fine grain readily disperse, it is difficult to make solution from the granular powder specimen. This has deterred the way to the automatic inspection of cellulose derivative solutions. Since further some water-soluble cellulose derivatives, for example, alkylcellulose, hydroxyalkylcellulose, hydroxyalkylalkylcellulose, are dissolved not in hot water but in cold water, their dissolution process is compelled to be complicated. To prepare the solution, first hot water is added to water-soluble cellulose derivative, then the mixture is forcibly stirred by means of rotary vanes, for instance, to fully gelate and disperse the cellulose. Then the recipient containing the mixture is immersed in the dissolution layer at 5°C or lower to dissolve the cellulose, and water is injected to make a solution of prescribed concentration. The water solution thus prepared forms the subject of a variety of measurements.

Conventionally we have had no such automatic measuring equipment as can obtain the solution and perform consistently to the last, the respective inspection items for the solution of cellulose derivatives. Because the preparation and measurement of solution was made manually for each inspection item, the work efficiency was poor. Same specimen was sometimes measured to differing values depending upon the inspector.

From the document EP-A-606 121 a solution preparing device is shown. In this device a specimen of a powdery product is divided into several subdivisions. Each subdivision is contained in a specimen recipient and is displaced parallel to the other subdivisions to a corresponding measuring means.

From the document FR-A-2 555 744 it is known to permanently attach an erasable writable memory (EPROM) to a sample carrier in order to correctly identify a sample included in the carrier and to store the results of a plurality of measurements. The same as stated with respect to the document FR-A-2 555 744 also applies to the document DE-A-43 06 563.

The further document "Chemometrics & Intelligent laboratory systems", Vol. 17, pp.123-127 deals with a robotic sample preparation in which a wide range of processes as a part of a robotic automatic analysis system is automatically performed.

The object of the invention is to provide an automatic measuring equipment in which the steps of preparing a cellulose derivative solution from powdered cellulose derivatives can be automatically performed.

The purpose of this invention is to supply an automatic measuring equipment that measures nonattendedly, namely, without any person attending, plural inspection items of physical properties of cellulose derivative solutions with excellent repeatability and high working efficiency.

The above-stated object is achieved by means of the combination of the features defined in the independent claim 1. Preferred embodiments of the automatic measuring equipment according to claim 1 are disclosed in the dependent claims 2 and 3, respectively.

In the following the invention is further illustrated by an embodiment with reference to the enclosed figures.
FIGURE 1 is a plan view illustrating an embodiment of the automatic measuring equipment that measures the physical properties of cellulose derivative solutions according to this invention.
FIGURE 2 is a perspective illustration that shows the essential portions of the automatic measuring equipment to which this invention applies.
FIGURE 3 is another perspective illustration that shows the solution preparing apparatus to be incorporated into the automatic measuring equipment by this invention.
FIGURE 4 is yet another perspective illustration that shows the viscosity measuring apparatus to be incorporated into the automatic measuring equipment by this invention.
FIGURE 5 is still another perspective illustration that shows the electrochemical analyzer to be incorporated into the automatic measuring equipment by this invention.
FIGURE 6 is diagrammatical plan view of the transmittance measuring apparatus to be incorporated into the automatic measuring equipment by this invention.
FIGURE 7 is another diagrammatical but side view of the image measuring apparatus to be incorporated into the automatic measuring equipment by this invention.

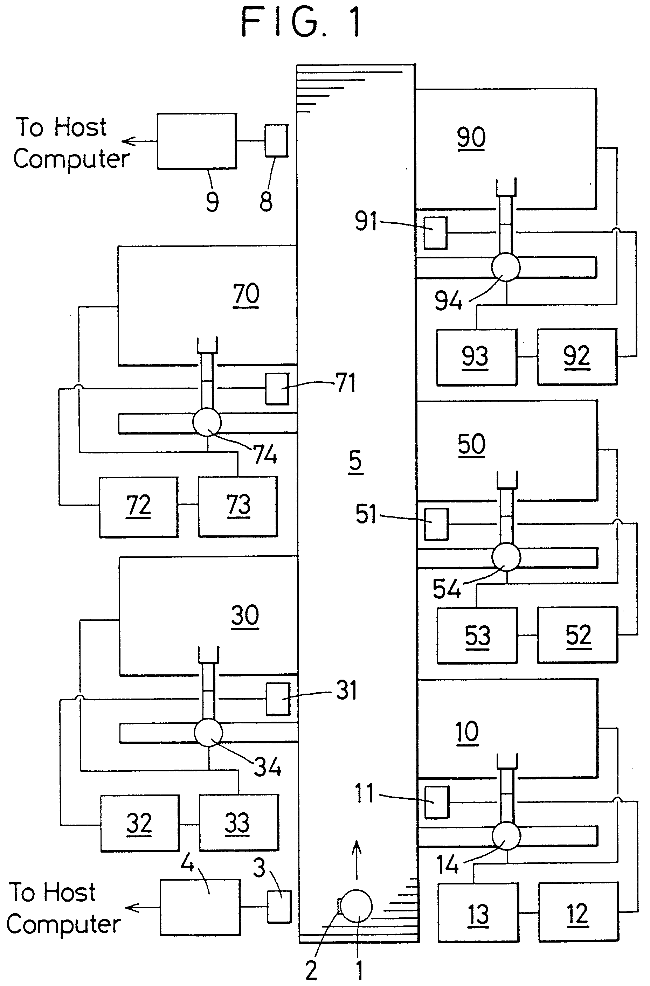

Referring now to FIGURE 1 that shows an embodiment of this invention made to achieve said objective, we attempt to describe the automatic measuring equipment that can measure the physical properties of cellulose derivative solutions to be manufactured according to this invention.

As is shown in FIGURE 1, the automatic measuring equipment by this invention, which has a device 10 that prepares the solution of fixed concentration from the specimen of cellulose derivatives, and the measuring devices 30, 50, 70, and 90 that measure the properties of the solution under plural inspection items, is characterized in that specimen of cellulose derivative is put into the equipment, that the specimen recipient 1 that displaces sequentially to the solution preparing device 10 and to the plural inspection/measuring apparatuses 30, 50, 70, and 90 is provided with an erasable write-in memory 2, that provided in the vicinity of the displacement starting point of the specimen recipient 1 are the input means 3 and 4 which write in the memory 2 the inspection item data and/or inspection condition data and that provided on the solution preparing device 10 and on the plural inspection/measuring apparatuses 30, 50, 70, and 90 respectively are the data reading devices 11/12, 31/32, 51/52, 71/72, and 91/92 that read out data from the memory 2 and the sequencers 13, 33, 53, 73 and 93 which gives instructions to the respective device and apparatuses 10, 30, 50, 70 , and 90 in terms of the inspection items and/or inspection conditions as read out from the data. The automatic measuring equipment in another embodiment of the automatic measuring equipment by this invention for the physical properties of cellulose derivative solutions is characterized in that the respective measuring apparatuses 30, 50, 70, and 90 are provided with the input means that write the inspection results into the memory 2, and that provided in the vicinity of the displacement end point of the specimen recipient 1 are the device 8 which reads in the inspection results from the memory 2 and means 9 to transfer said inspection results to host computer. The input means to write in the test results may be commonly used for that purpose with the devices 11/12, 31/32, 51/52, 71/72, and 91/92 that read out the data from the memory 2.

The material embodiment of the automatic measuring equipment for physical properties of cellulose derivative solutions by this invention is characterized in that said plural inspection/measuring apparatuses comprise the viscosity measuring apparatus 30, electrochemical analyzer 50, transmittance measuring apparatus 70, and the image analyzer 90.

The specimen recipient 1 that contains the specimen of cellulose derivative is provided with the erasable write-in memory 2 for the inspection item data and inspection condition data to be written into the memory 2 from the input means 3 and 4. These data being read out by the readers 11 and 12, the dissolving conditions of cellulose derivative are set at the sequencer 13, which gives instructions to dissolve, by the device 10, the cellular derivative in water and organic solvents, for instance, and the derivative thus dissolved is prepared into prescribed concentration and put in the specimen recipient 1. The specimen recipient 1 containing the solution of cellulose derivatives then displaces up to the inspection/measuring apparatus 30 (viscosity measuring apparatus in the case of this embodiment), the inspection item and condition data are read out of the memory 2 at the readers 31 and 32, and the inspection conditions will be set at the sequencer 33, which will give instructions to conduct by the automatic measuring apparatus 30, the inspection in accordance with the conditions. The specimen recipient 1 will further displace similarly to the inspection/measuring apparatus 50 (the electrochemical analyzer in this embodiment), inspection/measuring apparatus 70 (transmittance measuring apparatus in this embodiment),inspection/measuring apparatus 90 (image analyzer in this embodiment), wherein the inspection will be performed in the sequential order as specified in the inspection item and condition data stored in the memory 2. The results of the inspection/measurement conducted at the inspection/measurement apparatuses 30, 50, 70, and 90 are written into the memory 2 by the input means 31/32, 51/52, 71/72, and 91/92 (combinedly used with readers in this embodiment). Meanwhile the specimen recipient 1 displaces farther with the inspection results read by the reader 8 from the memory 2 and transferred to host computer.

Referring now to the drawings, we attempt to explain in detail the embodiment of this automatic measuring equipment for the physical properties of cellulose derivative solutions to which this invention applies. In this embodiment we will take, as an example, the physical properties of the solution made from water-soluble cellulose derivatives dissolved into water.

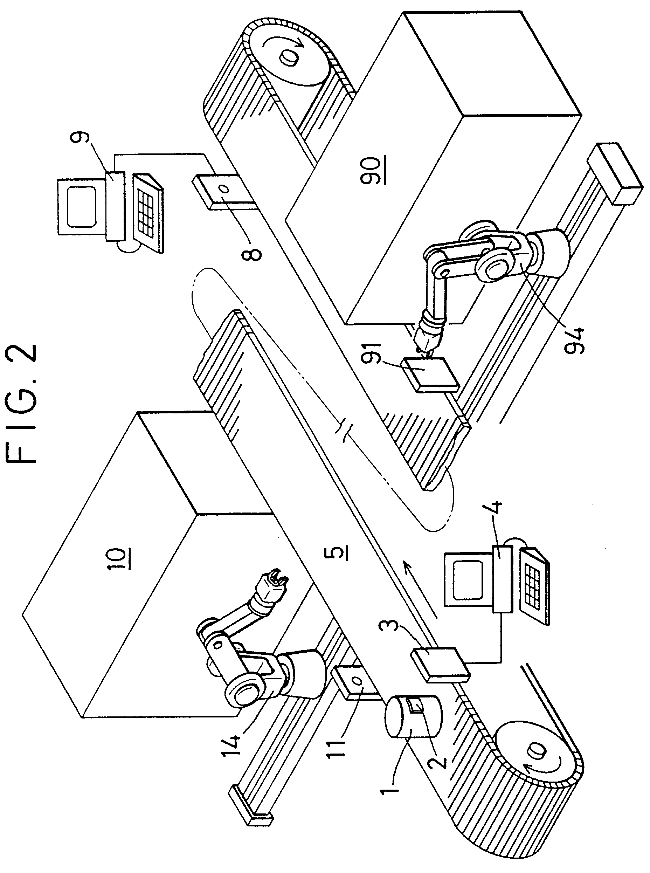

FIGURES 1 and 2 are the plan view of the embodiment of the automatic measuring equipment by this invention, and the perspective view of its essential portions, respectively. As shown in these drawings, provided in the center of the automatic measuring equipment is the conveyor 5, arranged on either side of which are the aqueous solution preparing device 10, viscosity measuring apparatus 30, electrochemical analyser 50, transmittance measuring apparatus 70, image analyzer 90, which are all arranged in this order. It is so designed that the specimen recipient 1 is displaced, by the conveyor 5, to the aqueous solution preparing device 10, viscosity measuring apparatus 30, electrochemical analyzer 50, transmittance measuring apparatus 70, image analyzer 90, in this order.

Attached on the specimen recipient 1 are the erasable write-in memory (RAM: Random Access Memory),called ID tag, the data receiver/transmitter and the chips for its control circuit. Provided at the point where the specimen recipient 1 begins to displace on the conveyor 5, are the terminal computer 4 that is the input means for writing the inspection item and condition data into the memory 2 as well as the receiver/transmitter circuit 3. Installed in the direct vicinity of the water solution preparing device 10 is the robot 14 that grasps and conveys the specimen recipient 1 brought forth on the conveyor 5 to said device 10 and manipulates this recipient 1 in the sequential order of driving the device 10. Further attached to this water solution preparing device 10 are the receiver/transmitter circuit 11 that receives the inspection item and condition data from the memory 2, the computer 12 that sets the dissolving conditions extracting required data from the data read out, and the sequencer 13 that gives the apparatus 10 the operational instructions under the dissolving conditions as set.

Installed in the nearest vicinity of the viscosity measuring apparatus 30 is the robot 34 that grasps and conveys the specimen recipient 1 displaced on the conveyor 5 to the viscosity measuring apparatus 30 and manipulates this same recipient 1 in the sequential order of driving the viscosity measuring apparatus 30. Provided further on this viscosity measuring apparatus 30 are the receiver/transmitter circuit 31 that receives, from the memory 2, the inspection item and condition data, and the computer 32 that extracts required data from the data as read out to set the measuring conditions, and the sequencer 33 that gives operational instructions to the viscosity measuring apparatus 30 under the measuring conditions as set. The receiver/transmitter circuit 31 is endowed with the function to transmit the inspection results measured at the viscosity measuring apparatus 30 and processed in the computer 12, the inspection result data thus transmitted being stored in the memory 2. Attached also on the electrochemical analyzer 50, transmittance measuring apparatus 70, and image analyzer 90 respectively are the robots 54, 74, and 94, the receiver/transmitter circuits 51, 71, and 91, the computers 52, 72, and 92, and finally the sequencers 53, 73, and 93.

Provided at the point where the specimen recipient 1 on the conveyor 5 has achieved its due displacement in the sequential order of the water solution preparing device 10, viscosity measuring apparatus 30, electrochemical analyzer 50, transmittance measuring apparatus 70, and image analyzer 90 are the receiver circuit 8 and terminal computer 9 that read out the inspection results from the memory 2. The receiver/transmitter circuit 8 consists of an ID antenna and its control circuit. The terminal computer 9 is connected to the host computer.

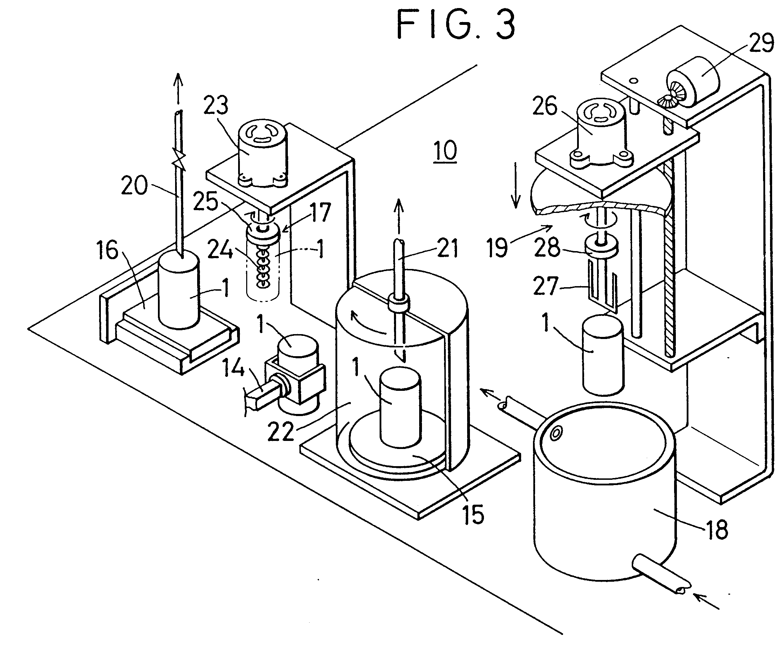

FIGURE 3 represents a perspective view of the aqueous solution preparing device 10 as one of the inspection/measuring apparatuses. The aqueous solution preparing device 10 has an electronic balance 15, a shaker 16, hot water stirrer 17, cooling tank 18, and cold water stirrer 19. The electronic balance 15 is covered with an open/close type hood 22. Inserted from the upper portion of this balance 15 is the cold water feed tube 21 that is connected with the supply source of cold water and discharges a very small quantity of cold water in linking with the weighing value of the electronic balance 15. The shaker 16 swings and sways violently the specimen recipient installed on it. Provided on the top of the shaker is the hot water feed tube 20 connected to the hot water supply source (not shown). The hot water stirrer 17 has the stirrer screw 24 that is connected to the geared motor 23, passing pivotally through the lid 25 fitted into the specimen recipient 1. In the cooling tank 18, water as heating medium is circulating, the cooling water of which is regulated to be held at 5°C. In the cooling stirrer 19, the screw 27 with agitating vanes is coupled with the geared motor 26. Said screw 27 passes pivotally through the lid 28 fitted into the specimen recipient 1. This screw, as a whole, is linked with the up and down mechanism composed of a screw rod and guide bar whose driving source is the motor 29.

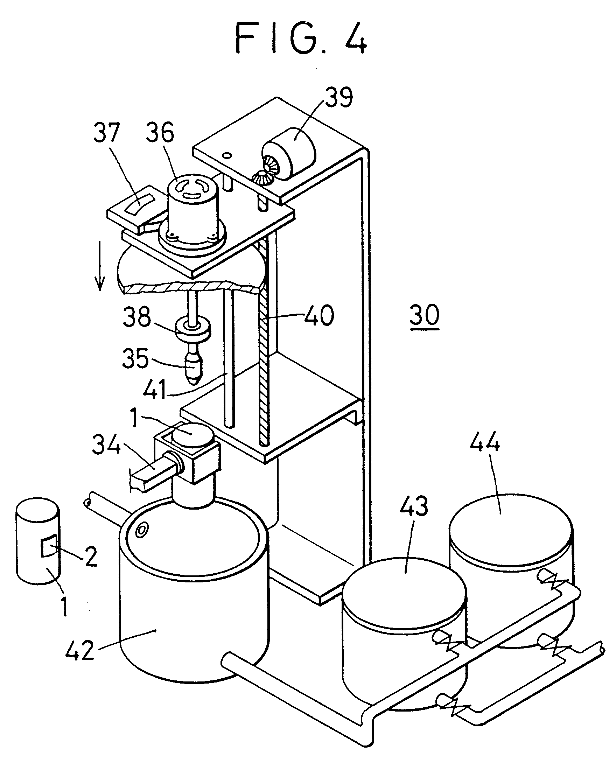

FIGURE 4 is a perspective view of the viscosity measuring apparatus 30 as one of the inspection/measuring apparatuses. The viscosity measuring apparatus 30 is in the form almost identical with the B type rotary viscosimeter as prescribed in the JIS (Japanese Industrial Standards). This apparatus serves to shape the rotor 35 into spindle form and reduces the swallowing-up of bubbles when it is immersed into the liquid specimen. The rotor 35, connected to the geared motor 36, passes pivotally through the lid 38 fitted into the specimen recipient 1. Connected to the motor 36 is a torque meter 37. The rotary system as a whole including these components takes the motor 39 as a driving source and is coupled with the up and down mechanism composed of the screw rod 40 and guide bar 41. Provided in the lower portion of this rotating system is a thermostatic chamber 42 to which are connected a water tank 43 held at constant temperature of 30°C which is higher than the measurement temperature and another water tank 44 held at constant temperature of 20°C, which is the measurement temperature.

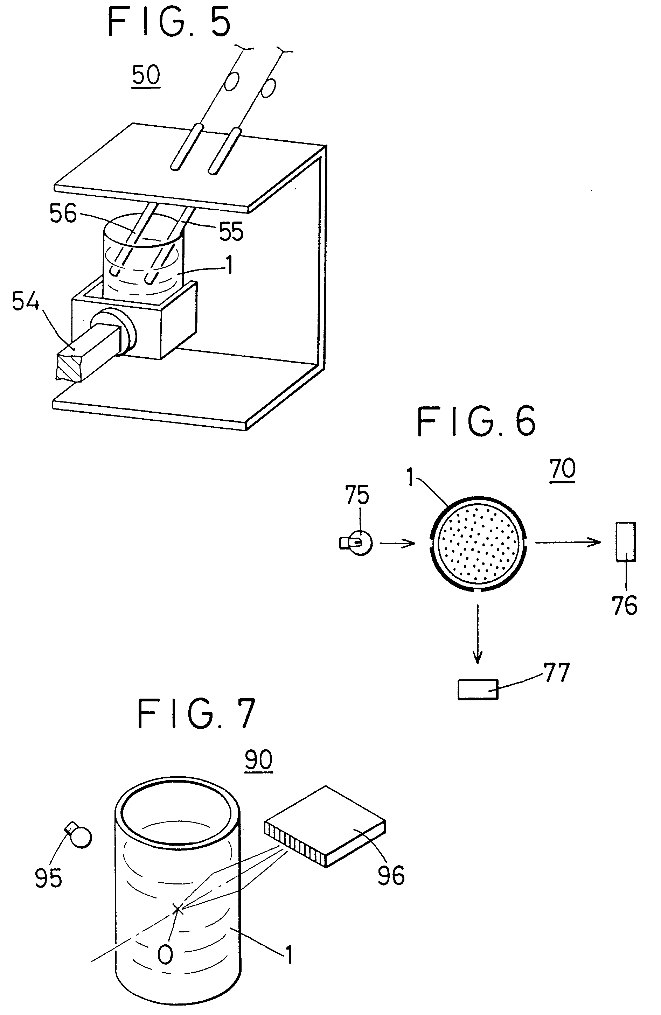

FIGURE 5 depicts a perspective view of the electrochemical analyzer 50 as one of the inspection/measuring apparatuses. The electrochemical analyzer 50 has the electrodes 55 and 56 on which voltage is supplied to measure the current. These electrodes 55 and 56 have been designed to be applied diagonally to the liquid surface so that no bubble may not be swallowed up when they are immersed into the liquid specimen.

FIGURE 6 is a diagrammatical layout (top view) of the transmittance measuring apparatus 70 as one of the inspection/measuring apparatuses. With the specimen recipient 1 in between, the light source 75 and the photoelectric converter 76 are arranged opposed to each other so that the rectilinearly propagating transmitted light may be measured. Arranged orthogonally to the direction of the rectilinear propagation is the photoelectric converter 77 so that one may measure the reflected rays that scatter due to the matter suspended in the liquid specimen in the recipient 1.

FIGURE 7 represents a diagrammatical lateral layout of the image analyzer 90 taken as one of the inspection/measuring apparatuses. Arranged in the upper diagonal direction of the specimen recipient 1 is the light source 95 and, in the horizontal opposite direction with the specimen recipient 1 in between, the cylindrical lens formed by the liquid specimen (curved surface of circular arc cylinder of the liquid formed in contact with the wall face of the cylindrical specimen recipient 1) is arranged at the position where the image of the vicinity of the central position O of the recipient 1 is formed, and CCD (Charge Coupled Device) sensor 96 with arrays is disposed in the horizontal direction.

The automatic measuring equipment that measures the physical properties of cellulose derivatives solution mentioned above operates as follows.

Powdered specimen of cellulose derivative, subject of the test, is put into the specimen recipient 1, while the terminal computer 4 receives such inspection items and conditions as required, together with the type or kinds of the specimen powder, manufacturing lot number and manufacturing date, month and year. The data is transferred from the terminal computer to the host computer. At the point where the recipient 1 containing the powdered specimen, set on the conveyor 5, begins to remove, the data input into the terminal computer 4 is written into the memory 2 provided on the specimen recipient 1 by means of the receiver/transmitter circuit 3. When the recipient 1 is carried, by the conveyor 5, up to the water solution preparing device 10 under these conditions, the receiver/transmitter circuit 11 reads out the data from the memory 2 and puts it into the computer 12, which sets the dissolving conditions based on the data and gives them to the sequencer 13, from where the operational instructions are given to the water solution preparing device 10, which will function as follows.

The robot 14 seizes up the specimen recipient 1, carries it to the electronic balance 15, and weighs the weight of the powdered specimen together with its tare (that is, the weight of the recipient) and the weight thus measured is input into the computer 12, where the weight of the specimen recipient 1 as read out from the memory 2 has been input as data, the heat volume of the powdered specimen is calculated out, and further the amount of the water required to make the water solution to the prescribed concentration is also calculated out. On the other hand, the recipient 1 containing the powdered specimen, after the completion of weighing, is taken up and carried, by the robot 14, to the shaker 16, where instructions are given from the sequencer 13, and the hot water a little less in volume than the required volume of water previously calculated is discharged gradually from the hot water feed tube to be put into the recipient 1. When the hot water permeates the powdered specimen as a whole, the shaker 16 is stopped with the specimen recipient 1 carried by robot 14 to the hot water stirrer 17, into which the stirring screw 24 will be inserted and locked up. Under these conditions (refer to the dotted line in FIGURE 3), the geared motor 23 is made to rotate, the hot water and powdered specimen are fully blended by the stirring screw 24 to swell up the specimen. When the stirring terminates, the specimen recipient 1 will be carried by the robot 14, and the stirring vanes 27 above the cooling tank 18 is inserted into the recipient 1 to fit it into the lid 28. If, after retiring the robot 14, the motor 29 is made to rotate, the recipient 1 comes down through the up and down mechanism composed of the screw rod and guide bar to be immersed into the cooling tank 18 held at 5°C. Then the geared motor 26 is made to rotate. If the stirring of the hot water and powdered specimen by means of the stirring vanes 27 is continued, the content of the specimen recipient 1 becomes cooler, and when the powdered specimen is cooled down to 5°C, it will be dissolved into water. If the motor 29 is made to turn reversely when the temporary dissolution is thus over, the specimen recipient 1 will go up. The recipient 1 is then detached from the lid 28 by the robot 14, and then carried again to the electronic balance 15. The liquid specimen will be achieved by replenishing a very small amount of water to make the water solution to prescribed concentration. The recipient 1 containing the specimen is then returned to the conveyor 5 by the robot 14, while the stirring screw 24 and the stirring vanes 27 are immersed and purified in the pure water in the recipient 1 to be transported by the robot 14.

The specimen recipient 1 is carried, by the conveyor 5, to the viscosity measuring apparatus 30. The receiver/transmitter circuit 31 reads out the data from the memory 2 and inputs it into the computer 32, which sets the viscosity measuring conditions based on the data and gives them to the sequencer 33, which issues operational instructions to the viscosity measuring apparatus 30. The specimen recipient 1 whose data has been read is carried by the robot 34 to the thermostatic tank 42 of the viscosity measuring apparatus 30, shown in FIGURE 4, to be immersed. To the thermostatic tank 42, water of 30°C, higher than the measurement temperature is sent from the water tank 43 to raise the temperature rising rate. When the temperature of the liquid specimen is lowered down nearly to 20°C, the measurement temperature, water at 20°C is sent from the water tank 44 to hold the liquid specimen at constant temperature. If, under these conditions, the motor 39 is made to rotate, the up and down mechanism composed of the screw rod 40 and guide bar 41 descends the rotor 35, motor 36 and torque meter 37, and the robot 35 is inserted in the specimen recipient 1 immersed in the thermostatic tank 52 to be fitted into the lid 38. The motor 36 is then made to rotate, and when the rotation becomes stable, the value of the toque meter 37 is taken into the computer 32, which will calculate the viscosity by adding to that value necessary calibration. The specimen recipient 1 containing the liquid specimen whose viscosity measurement is now over is returned by the robot 34 onto the conveyor 5, while the rotor 35 is immersed and purified in the pure water in the recipient 1 to be conveyed later by the robot 34.

The viscosity as calculated by the computer 32 is written from the receiver/transmitter circuit 31 into the memory 2 of the specimen recipient 1 to be thus returned to the conveyor 5. The specimen recipient 1 containing the liquid specimen is carried by the conveyor 5 to the electro-chemical analyzer 50. The data read out from the memory 2 by means of the receiver/transmitter circuit 51 is input into the computer 52, which will then set the conditions of the electrochemical analysis from that data to give them to the sequencer 53, which will give operational instructions to the electrochemical analyser 50. The recipient 1 whose data has now been read out is carried by the robot 54 to the electrochemical analyzer 50 as shown in FIGURE 5, and the electrodes 55 and 56 are held in the liquid specimen so that they may be immersed, to which voltage is applied to measure the current. Qualitative and quantitative analyses, and pH measurement are made of the inorganic matter in terms of the type of the electrodes 55 and 56, voltage and current. The results of these analyses are input into the computer 52. The recipient 1 including the liquid specimen whose electrochemical analysis is now over, is returned onto the conveyor 5 by means of the robot 54, while the electrodes 55 and 56 are immersed and purified in the pure water in the recipient 1 to be then transported by the robot 54.

The results of the analyses put in the computer 52 are written, through the receiver/transmitter circuit 51, into the memory 2 of the specimen recipient 1 now returned to the conveyor 5. The recipient 1 containing the liquid specimen is carried, by the robot 5, up to the transmittance measuring apparatus 70, with the data read out from the memory 2 by the receiver/transmitter circuit 71 to be input into the computer 72, which sets the transmittance measuring conditions from the data and give them to the sequencer 73, which will give the transmittance measuring apparatus 70 operational instructions. The recipient 1 whose data has been read out is carried by the robot 74 to the transmittance measuring apparatus 70 and mounted at the position shown in FIGURE 6. When the light source 75 is lit up in the transmittance measuring apparatus 70, a part of the light goes straight into the liquid specimen in the recipient 1 and the light thus transmitted is measured at the photoelectric converter 76. On the other hand, a part of the light from the light source 75 is scattered by the substance suspended in the liquid specimen in the recipient 1, repeatedly reflected on the outer and inner walls of the recipient 1, and a part of its component is measured at the photoelectric converter 77. The computer 72 performs a statistical corrective operation in terms of the quantity of light from the photoelectric converter 76 and 77 to calculate out the transmittance, turbidity and suspended (foreign) matter of the liquid specimen and stores these values. Thus the specimen recipient 1 containing the liquid specimen whose transmittance has now been measured is returned to the conveyor by the robot 74.

Such values as transmittance stored in the computer 72 are written, by the receiver/transmitter circuit 71, into the memory 2 of the recipient 1 now returned to the conveyor 5. The recipient containing the liquid specimen is then carried by the conveyor 5 up to the image analyzer 90. The data is read out from the memory 2 by the receiver/transmitter circuit 91 to be input into the computer 92, which will set the conditions of the image analysis from the data and gives them to the sequencer 93, which will give operational instructions to the image analyzer 90. The specimen recipient 1 whose data has now been read out is carried by the robot 94 to the image analyzer 90 and mounted on the position as shown in FIGURE 7. In the image analyzer 90, which is illuminated by the light source 95, the image of the substance floating freely near the central portion O of the specimen recipient 1 is formed into linear lines in the horizontal direction on the CCD sensor 96 by the cylindrical lens to be shaped by the contact of the liquid specimen with the wall face of the recipient 1. This image is scanned by the CCD sensor 96, and the data thereof is image-analyzed by the computer 92 to calculate the length of the suspended matter (nondissolved fiber).

The specimen recipient 1 containing the liquid specimen whose image analysis is now over is returned onto the conveyor 5 by the robot 94. Such data as the length of the nondissolved fiber calculated out by the computer 92 is written, by the receiver/transmitter circuit 91, into the memory 2 of the recipient 1 now returned to the conveyor 5.

The recipient 1 containing the liquid specimen whose inspections/measurements have now all over is further carried forward. The data of the inspection results as written into the memory 2 is read out by the receiver circuit 8 to be forwarded to the host computer through the terminal computer 9.

As has thus far been described in detail, the equipment that measures automatically physical properties of cellulose derivative solutions to which this invention applies allow to obtain solution from the derivatives and perform automatic and consistent measurement for the respective inspection items without any personnel attending to the measurement, and this with superb repeatability and high accuracy in the inspection. Even if a unit in the equipment fails, the equipment as a whole can be operated except this unit, thus materializing an extremely efficient automatized equipment.

Provided is an equipment that is the most appropriate for the automatic measurement of the physical properties of cellulose derivative solutions. The automatic measuring equipment has a device 10 that prepares the specimen of cellulose derivatives to prescribed concentration, and the apparatuses 30, 50, 70, and 90 that measure the physical properties of said solution under plural inspection items. An erasable write-in memory 2 is provided on the specimen recipient 1 containing the specimen of cellulose derivatives that displaces to the solution preparing device 10, to plural inspection/measuring apparatuses 30 and others in sequential order, and provided in the vicinity of the displacement starting point of the specimen recipient 1 are the input means 3 and 4 that write the inspection data into the memory 2, and the solution preparing device 10 and plural measuring apparatuses 30 and others are provided with the apparatuses 30 and others are provided with the devices 11 and 12 that read in data from the memory 2 as well as the sequencers 13, 33, and others that give instructions to the respective devices and apparatuses 10, 30 and others depending upon the inspection data thus read out.

## Claims

1. An automatic measuring equipment for measuring physical properties of a cellulose derivative solution comprising
a solution preparing means (10) for preparing a cellulose derivative solution from a powdered cellulose derivative;
means (30, 50, 70, 90) for measuring physical properties with respect to plural inspection items;
a specimen recipient (1), which is provided with an erasable write-in memory (2), for holding the powdered cellulose derivative or the solution of the same, wherein the specimen recipient (1) can be moved to the solution preparing means (10) and the measuring means (30, 50, 70, 90);
an input means (3, 4) for writing inspection item data and condition data into the memory (2), which is disposed in the vicinity of the starting point of moving the specimen recipient (1) ;
means (11, 12, 31, 32, 51, 52, 71, 72, 91, 92) for reading the data from the memory (2), which are disposed in the vicinity of each of the solution preparing means (10) and the measuring means (30, 50, 70, 90); and
sequencers (13, 33, 53, 73, 93) for instructing the solution preparing means (10) and the measuring means (30, 50, 70, 90) in terms of the data read by the reading means (11, 12, 31, 32, 51, 52, 71, 72, 91, 92); wherein
said solution preparing means (10) is a means for automatically preparing the cellulose derivative solution of a prescribed concentration from the powdered cellulose derivative and includes
- an electric balance (15) for weighing the powdered cellulose derivative;
- a shaker (16) for violently swinging and swaying the specimen recipient (1) installed thereon, while the powdered cellulose derivative is permeated by a hot solvent;
- a hot solvent stirrer (17) for fully blending the mixture of the hot solvent and the powdered cellulose derivative; and
- a cooling stirrer (19) for continuing the stirring of the mixture of the hot solvent and the powdered cellulose derivative, while the specimen recipient (1) is cooled by a cooling means (18).

2. An automatic measuring equipment according to claim 1, characterized by
input means (11, 12, 31, 32, 51, 52, 71, 72, 91, 92) for writing inspection results from the measuring means (30, 50, 70, 90) into the memory (2) of the specimen recipient (1), which are disposed in the vicinity of each of the measuring means (30, 50, 70, 90); and
a means (9) for transferring the inspection results from the memory (2) to a host computer, which is disposed in the vicinity of the end point of moving the specimen recipient (1).

3. An automatic measuring equipment according to claim 1 or 2, characterized in that at least two of the plural measuring means (30, 50, 70, 90) are selected from a viscosity measuring means (30), an electrochemical analyzing means (50), a transmittance measuring means (70) and an image analyzing means (90).

## Patentansprüche

1. Automatische Meßanlage zur Messung physikalischer Eigenschaften einer Zellulosederivatlösung mit
einer Lösungsherstellungseinrichtung (10) zur Herstellung einer Zellulosederivatlösung aus einem pulverförmigen Zellulosederivat;
Einrichtungen (30, 50, 70, 90) zur Messung physikalischer Eigenschaften bezüglich einer Vielzahl von Prüfungsgegenständen;
einer mit einem löschbaren Einlesespeicher (2) versehenen Probenaufnahme (1) zum Halten des pulverförmigen Zellulosederivats oder dessen Lösung, wobei die Probenaufnahme (1) zu der Lösungsherstellungseinrichtung (10) und den Meßeinrichtungen (30, 50, 70, 90) bewegbar ist;
einer Eingabeeinrichtung (3, 4) zum Schreiben von Prüfungsgegenstandsdaten und Zustandsdaten in den Speicher (2), der in der Umgebung des Bewegungsstartpunkts der Probenaufnahme (1) angeordnet ist;
Einrichtungen (11, 12, 31, 32, 51, 52, 71, 72, 91, 92) zum Lesen der Daten von dem Speicher (2), die in der Umgebung von jeweils der Lösungsherstellungseinrichtung (10) und den Meßeinrichtungen (30, 50, 70, 90) angeordnet sind; und
Datenzuordnern (13, 33, 53, 73, 93) zum Instruieren der Lösungsherstellungseinrichtung (10) und der Meßeinrichtungen (30, 50, 70, 90) bezüglich der von den Leseeinrichtungen (11, 12, 31, 32, 51, 52, 71, 72, 91, 92) gelesenen Daten; wobei
die Lösungsherstellungseinrichtung (10) eine Einrichtung zur automatischen Herstellung der Zellulosederivatlösung einer vorbestimmten Konzentration aus dem pulverförmigen Zellulosederivat ist und aufweist
eine elektrische Waage (15) zum Wiegen des pulverförmigen Zellulosederivats;
eine Schütteleinrichtung (16) zum zwangsweisen Schwenken und Schwingen der darauf installierten Probenaufnahme (1), während das pulverförmige Zellulosederivat von einem heißen Lösungsmittel durchsetzt wird;
einen Heißlösungsmittelrührer (17) zum vollständigen Durchmischen des Gemisches aus dem heißen Lösungsmittel und dem pulverförmigen Zellulosederivat; und
einen Kühlrüher (19) zum Fortsetzen des Rührens der Mischung des heißen Lösungsmittels und des pulverförmigen Zellulosederivats, während die Probenaufnahme (1) mittels einer Kühleinrichtung (18) gekühlt wird.

2. Automatische Meßanlage nach Anspruch 1, gekennzeichnet durch
in der Umgebung jeder der Meßeinrichtungen (30, 50, 70, 90) angeordnete Eingabeeinrichtungen (11, 12, 31, 32, 51, 52, 71, 72, 91, 92) zum Schreiben von Prüfungsergebnissen von der Meßeinrichtungen (30, 50, 70, 90) in den Speicher (2) der Probenaufnahme (1); und
eine Einrichtung (9) zum Transferieren der Prüfungsergebnisse von dem Speicher (2) zum einem Hostcomputer, der in der Umgebung des Bewegungsendpunktes der Probenaufnahme (1) angeordnet ist.

3. Automatische Meßanlage nach Anspruch 1 oder 2, dadurch gekennzeichnet, daß zumindest zwei aus der Vielzahl von Meßeinrichtungen (30, 50, 70, 90) aus einer Viskositätsmeßeinrichtung (30), einer elektrochemischen Analyseeinrichtung (50), einer Durchlässigkeitsmeßeinrichtung (70) und einer Bildanalyseeinrichtung (90) ausgewählt sind.

## Revendications

1. Equipement de mesure automatique pour la mesure des propriétés physiques d'une solution de dérivé de cellulose comprenant
un moyen de préparation d'une solution (10) pour préparer une solution de dérivé de cellulose à partir d'un dérivé de cellulose en poudre;
des moyens (30, 50, 70, 90) pour la mesure des propriétés physiques concernant plusieurs éléments de contrôle ;
un récipient d'échantillon (1) qui est muni d'une mémoire effaçable pour l'entrée d'écritures (2), pour contenir le dérivé de cellulose en poudre ou la solution de celui-ci, dans lequel le récipient d'échantillon (1) peut être déplacé vers le moyen de préparation d'une solution (10) et les moyens de mesure (30, 50, 70,90);
un moyen d'entrée de données (3, 4) pour écrire les données des éléments de contrôle et les données des conditions dans la mémoire (2), lequel est disposé au voisinage du point de départ du déplacement du récipient d'échantillon (1) ;
des moyens (11, 12, 31, 32, 51, 52, 71, 72, 91, 92) pour lire les données dans la mémoire (2), lesquels sont disposés au voisinage de chacun du moyen de préparation d'une solution (10) et des moyens de mesure (30, 50, 70, 90) ; et
des séquenceurs (13, 33, 53, 73, 93) pour instruire le moyen de préparation d'une solution (10) et les moyens de mesure (30, 50, 70, 90) en termes des données lues par le moyen de lecture (11, 12, 31, 32, 51, 52, 71, 72, 91, 92) ; dans lequel
ledit moyen de préparation d'une solution (10) est un moyen pour préparer automatiquement la solution de dérivé de cellulose d'une concentration prescrite à partir du dérivé de cellulose en poudre et comprend
- une balance électrique (15) pour peser le dérivé de cellulose en poudre ;
- un secoueur (16) pour balancer et faire aller et venir violemment le récipient d'échantillon (1) installé sur son dessus alors que le dérivé de cellulose en poudre est perméé par un solvant chaud ;
- un agitateur de solvant chaud (17) pour mélanger complètement le mélange du solvant chaud et du dérivé de cellulose en poudre ; et
- un agitateur de refroidissement (19) pour continuer l'agitation du mélange du solvant chaud et du dérivé de cellulose en poudre alors que le récipient de l'échantillon (1) est refroidi par un moyen de refroidissement (18).

2. Equipement de mesure automatique selon la revendication 1, caractérisé par
des moyens d'entrée de données (11, 12, 31, 32, 51, 52, 71, 72, 91, 92) pour écrire les résultats de contrôle à partir des moyens de mesure (30, 50, 70, 90) dans la mémoire (2) du récipient d'échantillon (1), lesquels sont disposés au voisinage de chacun des moyens de mesure (30, 50, 70, 90) ; et
un moyen (9) pour transférer les résultats de contrôle à partir de la mémoire (2) vers un ordinateur central, lequel est disposé au voisinage du point final du déplacement du récipient d'échantillon (1).

3. Equipement de mesure automatique selon la revendication 1 ou 2, caractérisé en ce qu'au moins deux des nombreux moyens de mesure (30, 50, 70, 90) sont choisis parmi un moyen de mesure de la viscosité (30), un moyen d'analyse électrochimique (50), un moyen de mesure du facteur de transmission (70) et un moyen d'analyse d'image (90).
